Europäisches Patentamt

European Patent Office

Office européen des brevets

(19)

(11) Numéro de publication : **0 089 266**
**B2**

(12) # NOUVEAU FASCICULE DE BREVET EUROPÉEN

(45) Date de publication du nouveau fascicule du brevet :
30.08.89

(21) Numéro de dépôt : 83400445.9

(22) Date de dépôt : 04.03.83

(51) Int. Cl.⁴ : **C 12 P 21/00**, A 61 K 35/74,
A 61 K 37/02// C12R1/22,
C12R1/43, C12R1/15

(54) **Procédé de préparation de protéoglycanes immunostimulants inducteurs d'interféron, protéoglycanes obtenus et médicament les contenant.**

(30) Priorité : 09.03.82 FR 8203921

(43) Date de publication de la demande :
21.09.83 Bulletin 83/38

(45) Mention de la délivrance du brevet :
19.06.85 Bulletin 85/25

(45) Mention de la décision concernant l'opposition :
30.08.89 Bulletin 89/35

(84) Etats contractants désignés :
AT BE CH DE FR GB IT LI LU NL SE

(56) Documents cités :
EP--A-- 0 013 851
EP--A-- 0 049 182
FR--A-- 2 088 112
FR--A-- 2 171 907
FR--A-- 2 248 025
FR--A-- 2 315 945
FR--A-- 2 378 855
FR--A-- 2 405 298
Proceedings of an International Symposium, Osaka,
Excerpta Medica, 1982, p. 261-269

(73) Titulaire : **PIERRE FABRE S.A.**
**125, rue de la Faisanderie**
**F-75116 Paris (FR)**

(72) Inventeur : **Dussourd d'Hinterland, Lucien**
**Domaine de Plombières Avenue de Lautrec**
**F-81100 Castres (FR)**
Inventeur : **Normier, Gérard**
**23 rue Francis Poullenc**
**F-81100 Castres (FR)**
Inventeur : **Pinel, Anne-Marie**
**22 rue des Capucins**
**F-81100 Castres (FR)**

(74) Mandataire : **Martin, Jean-Jacques et al**
**Cabinet REGIMBEAU 26, Avenue Kléber**
**F-75116 Paris (FR)**

EP 0 089 266 B2

Jouve, 18, rue St-Denis, 75001 Paris, France

## Description

La présente invention concerne un procédé de préparation d'une fraction protéoglycanique isolée de membranes bactériennes ainsi que la fraction obtenue et son utilisation comme immunostimulant, notamment pour l'activation des cellules N.K. (Natural Killer) connues pour leur pouvoir cytctoxique vis-à-vis des cellules tumorales.

Différents documents mentionnent la préparation de divers fractions glycoprotéïques ou protéoglycaniques de bactéries. La demande de brevet EP-A-O 049 182 décrit des procédés permettant de préparer des protéoglycanes immunostimulants.

Les extraits du symposium qui s'est tenu à OSAKA en Juillet 1981, notamment les pages 261 à 265 et 266 à 269, décrivent également des procédés de préparation des protéoglycanes.

Un poster présenté à VIENNE le 14 Septembre 1981 mentionne des fractions du même type que précédemment.

Enfin, les brevets FR 2 171 907, FR 2 088 112, FR 2 043 475 décrivent des extraits bactériens de nature glycoprotéïque.

Le brevet français n° 78 35649 (EP-A-13 851) déposé le 19 décembre 1978 au nom de la Demanderesse décrivait la préparation de protéoglycanes menbranaires détoxifiés à partir de bactéries gram négatif et leur utilisation comme adjuvant dans des vaccins.

La présente invention décrit, à partir de ce type de protéoglycanes membranaires provenant notamment d'une souche de Klebsiella pneumoniae, l'isolement d'une fraction protéoglycanique possédant des propriétés immurostimulantes dont la plus remarquable est d'activer fortement la stimulation des cellules N.K.

Le procédé selon la présente invention, permettant de préparer des protéoglycanes membranaires bactériens ayant une activité immunostimulante, notamment inductrice d'interféron, à partir de protéoglycanes membranaires solubles d'une souche de bactérie gram négatif, est caractérisé en ce que :

— on traite en milieu aqueux les protéoglycanes membranaires bruts isolés de ladite souche par une base ou un hypobromite et on récupère la phase aqueuse contenant les protéoglycanes solubles,

— puis les protéoglycanes membranaires solubles sont hydrolysés par le lysozyme, et

— on sépare du produit d'hydrolyse les protéoglycanes ayant un poids moléculaire compris entre 200 000 et 400 000.

Les protéoglycanes membranaires solubles utilisés comme produits de départ dans le procédé selon l'invention sont préparés, de préférence, selon les procédés décrits dans le brevet français n° 78 35649. . Dans le procédé du brevet français n° 78 35649, l'étape essentielle consiste à traiter en milieu aqueux les protéoglycanes membranaires bruts, séparés par l'un quelconque des procédés connus, par une base ou un hypobromite et à récupérer la phase aqueuse contenant les protéoglycanes solubles.

D'autres caractéristiques de ce procédé pourront être trouvées dans le brevet précédent dont les enseignements sont incorporés dans la présente description à titre de référence.

La séparation des protéoglycanes ayant un poids moléculaire compris entre 200 000 et 400 000 qui constituent la fraction active selon l'invention peut être effectuée par un procédé quelconque, en particulier en utilisant une chromatographie sur tamis moléculaire, par exemple sur une colonne de gel Sépharose CL2 B.

Le traitement par le lysozyme hydrolyse les liaisons N-acétylmuramyl-β-(1-4)-N-acétylglucosamine des protéoglycanes en réduisant ainsi le poids moléculaire de ces fractions. La durée de cette hydrolyse est de préférence comprise entre 10 minutes et 1 heure et, en général, de l'ordre de 30 minutes. Avant de soumettre le produit d'hydrolyse au fractionnement chromatographique, il est intéressant d'éliminer le lysozyme et les différents sels ou composés présents.

Les protéoglycanes solubles sont, de préférence, délipidés, par exemple à l'aide d'un ou plusieurs solvants des lipides, avant d'être soumis à l'action du lysozyme.

Parmi les bactéries gram négatif susceptibles d'être mises en oeuvre, il faut citer plus particulièrement Klebsiella pneumoniae, Serratia marcescens et Escherichia coli et, tout spécialement, la souche de Klebsiella pneumoniae qui est une souche non capsulée, isolée par le Centre d'Immunologie et de Biologie PIERRE FABRE et déposée à la Collection Nationale de Culture de Microorganismes (CNCM) sous le n° 145-I-IP.

Les protéoglycanes ainsi obtenus ont les caractéristiques physico-chimiques ci-après :

| | |
|---|---:|
| — poids moléculaire compris entre | 200 000 et 400 000 |
| — teneur en hexoses, | 8 et 10 % |
| — teneur en galactose, | 22 et 28 % |
| — teneur en hexosamines, | 3 et 6 % |
| — teneur en acides uroniques, | 4 et 6 % |
| — teneur en protéines, | 35 et 50 % |

La présente invention concerne également l'utilisation de ces protéoglycanes à titre de médicament.

En effet, ces protéoglycanes présentent de précieuses propriétés immunostimulantes.

Ces propriétés immunostimulantes ont été mises en évidence par voie injectable et par voie orale chez la souris.

Ainsi, il a été mis en évidence pour ces produits une forte activation des cellules N. K. significative $(P > 0,01)$ sur un lymphome induit par le virus de Moloney chez la souris. Cette action est inhibée par un anti-interféron ($\alpha$-IF), ce qui montre que l'activation des cellules N. K. par cette fraction est due à un effet inducteur d'interféron.

Chez les animaux jeunes, les protéoglycanes stimulent à la fois des cellules N. K. et pré-N. K.

On observe également une stimulation de la synthèse de DNA in vitro par les cellules spléniques de souris, de façon proportionnelle à la dose, et une potentialisation de la réponse anticorps précoce à la sérumalbumine bovine (BSA).

En outre, une très forte stimulation des macrophages est mise en évidence dans les cinq systèmes suivants :

— chimiluminescence des granulocytes humains in vitro,
— phagocytose in vitro de la protéine A marquée par 125 I,
— étude de la nature des macrophages activé (Ia$^+$ ou Ia$^-$),
— test d'élimination du carbone colloïdal in vivo,
— test de protection contre Candida albicans.

Dans tous les systèmes étudiés, des modifications significatives de l'activité cellulaire sont observées qui se traduisent in vivo par une augmentation marquée de l'élimination du carbone et une résistance accrue à l'infection par Candida albicans. Il a été mis en évidence un impact considérable sur les premiers stades de la phagocytose de granulocytes humains, par un test de chimiluminescence. Sur les macrophages par une mesure in vitro après activation in vivo, augmentation des propriétés phagocytaires en induisant simultanément un phénotype plus primitif.

Les exemples suivant sont destinés à illustrer un procédé de préparation de protéoglycanes selon l'invention.

## Exemple 1

### Préparation de protéoglycanes membranaires solubles

#### a) Isolement des protéoglycanes membranaires bruts

La biomasse de la souche de Klebsiella pneumoniae 145-I-IP est dispersée dans un tampon Tris-HCl 0,01 M, pH 7,0, contenant NaCl 0,15 M glacé puis soumise à un broyage mécanique destiné à rompre les parois cellulaires.

Le lysat bactérien est clarifié par une centrifugation de 10 minutes à 7 500 g puis le surnageant est centrifugé pendant 45 minutes à 30 000 g.

Le culot est dispersé dans une solution aqueuse de NaCl 0,15 M. La suspension est à nouveau clarifiée 10 minutes à 7 500 g puis centrifugée 45 minutes à 30 000 g.

· Le culot est repris dans l'eau distillée puis à nouveau soumis à un cycle de centrifugation à 7 500 g puis 30 000 g.

Le culot de protéoglycanes membranaires bruts est alors repris dans 1/4 du volume initial d'eau distillée, la suspension est clarifiée 10 minutes à 7 500 g et le surnageant est lyophilisé.

#### b) Extraction des protéoglycanes membranaires solubles

Les protéoglycanes membranaires bruts lyophilisés sont dispersés dans NaOH (0,1 M) puis soumis à une hydrolyse ménagée pendant 1 heure à 56 °C. Après refroidissement, la suspension est neutralisée par HCl dilué puis dialysée 24 heures contre de l'eau distillée. La suspension est alors clarifiée par centrifugation 45 minutes à 30 000 g puis le surnageant est filtré sur membrane 0,22 μ. Le filtrat est lyophilisé.

## Exemple 2

### Préparation de la fraction immunostimulante

#### a) Délipidation des protéoglycanes membranaires solubles

Le lyophilisat de protéoglycanes membranaires solubles obtenu à l'exemple 1 est délipidé par une première extraction de 2 heures à 25 °C dans un mélange chloroforme/méthanol (2/1).

Après essorage sur un verre fritté n° 4, le résidu est extrait 2 heures à 25 °C dans un mélange éther/éthanol (1/3).

Après essorage sur verre fritté n° 4, le résidu est desséché sous vide.

b) Traitement par le lysozyme

Le résidu sec de protéoglycanes solubles délipidés est dissous dans du tampon Tris-HCl 0,015 M, pH 0,8, contenant EDTA, $Na_2$ 0,008 M et 80 mg/l de lysozyme.

Après 30 minutes d'incubation à 25 °C, la solution est chromatographiée sur une colonne de Biogel $P_{30}$ dans l'eau distillée.

Le premier pic élué au volume d'exclusion est recueilli puis dialysé contre de l'eau distillée.

Après filtration sur membrane 0,22 μ, le filtrat est lyophilisé.

c) Fractionnement chromatographique

Le lyophilisat précédent est dissous dans du tampon Tris-HCl 0,01 M, pH 7,0, puis soumis à un fractionnement par chromatographie de tamisage moléculaire sur une colonne de gel Sépharose CL 2 B, équilibrée avec le même tampon.

Les fractions correspondant au pic contenant l'activité immunostimulante ayant un poids moléculaire compris entre 200 000 et 400 000 sont regroupées puis dialysées contre de l'eau distillée.

Le dialysat est stérilisé par filtration sur membrane 0,22 μ puis lyophilisé stérilement.

Le lyophilisat constitue la fraction protéoglycanique immunostimulante que sera utilisée dans les exemples suivants.

La fraction protéoglycanique telle qu'elle est isolée en un pic homogène sur Sépharose CL 2 B présente les caractères analytiques moyens suivants :

| | |
|---|---|
| — poids moléculaire, | ($\approx$300 000) |
| — teneur en hexoses, | 9,3 % |
| — teneur en galactose, | 25,2 % |
| — teneur en hexosamines, | 4,5 % |
| — teneur en acides uroniques, | 5,3 % |
| — teneur en protéines, | 42,5 % |
| — teneur en ARN, | < 0,05 % |
| — teneur en ADN, | < 0,02 % |
| — teneur en acides gras, | $\ll$ 1 % |

Les exemples suivants sont destinés à mettre en évidence les propriétés immunostimulantes des protéoglycanes obtenus dans l'exemple 2.

L'ensemble des tests N. K. décrit ci-après ont été conduits selon le protocole général décrit dans Kiessling R. et Wigzell H., Immunol. Rev. 44, 165 (1979).

Exemple 3

Activation des cellules N. K. par voie injectable

Animaux : souris CBA/J de 4 mois (mâles)

Cellules cibles :
— Yac-1 sensibles aux N. K. (lymphome induit par le virus de Moloney chez la souris).

Protocole

Les souris reçoivent par voie intra-péritonéale 15 μg de la fraction protéoglycanique dans 0,2 ml de PBS.

Le test est effectué 24 heures après.

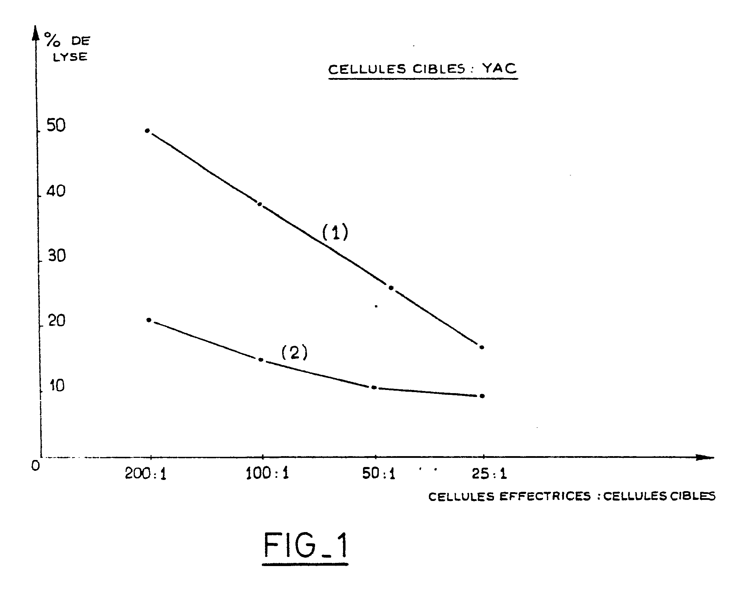

Les résultats mesurés sont rassemblés sur le graphique de la figure 1.

Sur ces graphiques, on a représenté le pourcentage de lyse des cellules cibles en fonction du rapport en nombre de cellules effectrices (c'est-à-dire des cellules N. K.) par rapport aux cellules cibles.

Sur la figure 1, la courbe 1 correspond à des animaux traités alors que la courbe 2 correspond à un groupe témoin.

On constate une forte activation des cellules N. K. chez les animaux traités par rapport aux animaux témoins.

La réponse est significative ($P > 0,01$) par rapport aux valeurs témoins dans ce test.

Exemple 4

Activation des cellules N. K. par voie orale

4

Animaux : souris CBA/H

Cellules cibles :
— cellules YAC sensibles aux N. K. (Lymphome Moloney)
— cellules P 815 insensibles aux N. K. (DBA/2 mastocytome).

Protocole

Les souris ont reçu chaque jour par voie orale 1 dose de 15 μg de fraction protéoglycanique pendant 14 jours ; une dose identique de 15 μg est ensuite administrée également par voie orale le 18ème jour.

Résultats

a) Cellules YAC (sensibles)

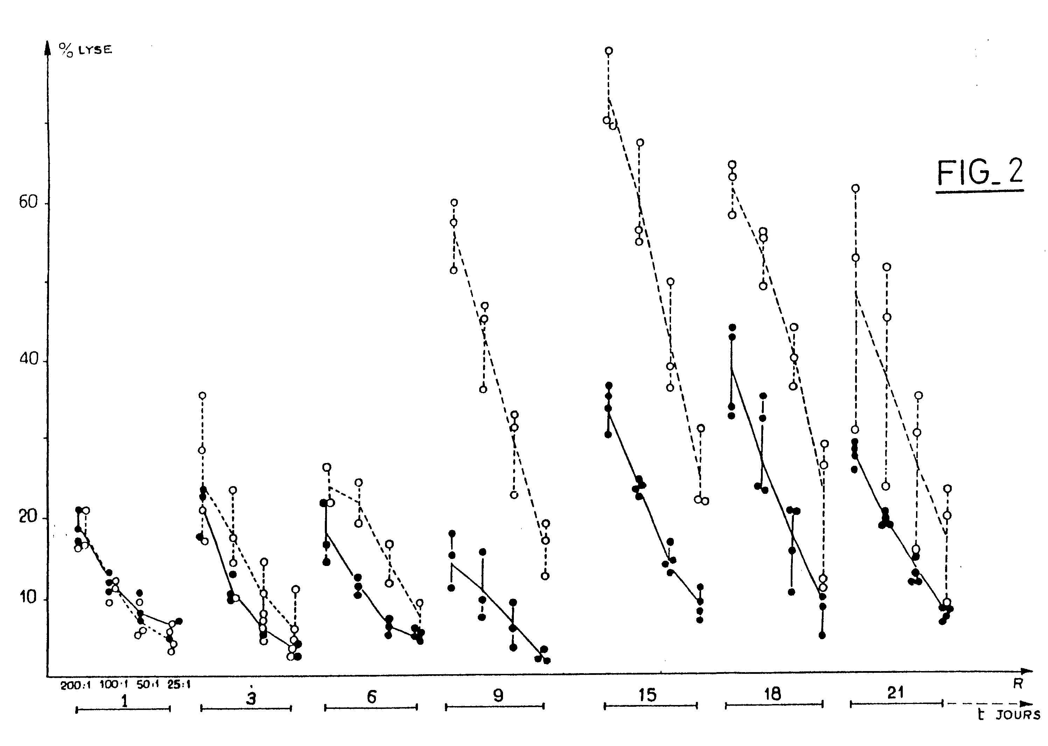

Les résultats mesurés sont représentés sur le graphique de la figure 2.

Sur ce graphique, on a représenté le pourcentage de lyse des cellules cibles en fonction du rapport entre les cellules effectrices et les cellules cibles mais à différentes époques. Ainsi on a indiqué en t le nombre de jours écoulés depuis le début du traitement et pour chaque jour on a mesuré le pourcentage de lyse à quatre dilutions différentes : 200/1, 50/1 et 25/1. Les points représentent chacun la moyenne d'un certain nombre d'essais et on a laissé apparaître les résultats observés pour trois groupes d'expériences. Les points blancs représentent les résultats observés pour les animaux traités, les points noirs pour les animaux témoins.

Les résultats sont extrêmements nets, on constate une augmentation hautement significative de l'activité N. K. Cette activité qui est détectable dès le 3ème jour devient très importante au 6ème et au 9ème jour et se maintient à un niveau très élevé pendant toute la durée de l'expérimentation (21 jours).

b) Cellules P 815 (non sensibles)

Les résultats observés montrent que contrairement au test N. K. positif utilisant comme cible des cellules YAC, il n'y a pas de cytotoxicité significative décelable à aucun moment de l'expérimentation.

Exemple 5

Vérification de la génération d'interféron par la fraction protéoglycanique

Animaux : souris CBA/J de 5 mois (mâles)

Cellules cibles :
— YAC-1 sensibles aux N. K.
— Nulli Teratoma sensibles aux N. K.

Protocole

Le test N. K. est fait par le protocole habituel avec des animaux recevant la fraction protéoglycanique seule ou avec l'anti-interféron (α-IF).

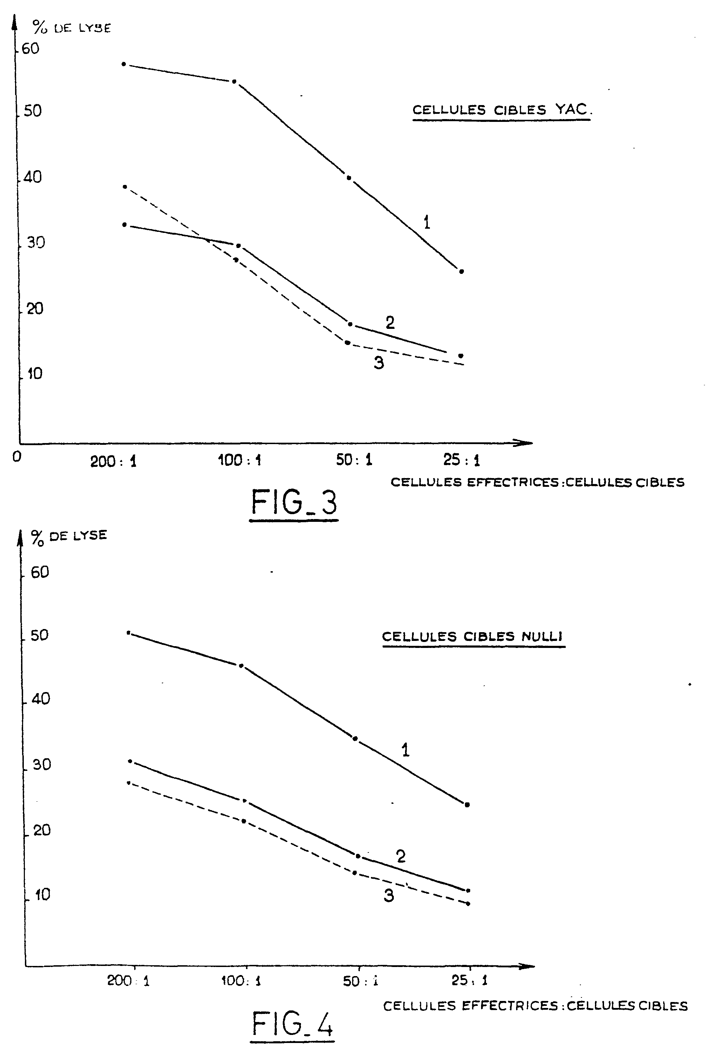

Les résultats observés sont rassemblés sur les graphiques des figures 3 et 4 établies comme cela est indiqué dans l'exemple 3.

Le graphique de la figure 3 rassemble les résultats observés sur les cellules cibles YAC.
— la courbe 1 correspond aux animaux traités,
— la courbe 2 correspond aux animaux non traités,
— la courbe 3 correspond aux animaux traités à la fois par les protéoglycanes et l'anti-interféron.

Le graphique de la figure 4 représente le même type de résultats mais pour des cellules cibles Nulli.

Ces résultats indiquent clairement que la fraction protéoglycanique active très fortement les cellules N. K. par l'intermédiaire d'une génération d'interféron.

**Revendications**

1. Procédé de préparation de protéoglycanes membranaires bactériens ayant une action inductrice d'interféron à partir des protéoglycanes membranaires solubles d'une souche de Klebsiella pneumoniae non capsulée, caractérisé en ce que :
— on traite en milieu aqueux les protéoglycanes membranaires bruts isolés de ladite souche par une base ou un hypobromite et on récupère la phase aqueuse contenant les protéoglycanes solubles,
— puis les protéoglycanes membranaires solubles sont hydrolysés par le lysozyme, et

— on sépare du produit d'hydrolyse les protéoglycanes ayant un poids moléculaire compris entre 200 000 et 400 000.

2. Procédé selon la revendication 1, caractérisé en ce qu'on sépare les protéoglycanes par chromatographie sur tamis moléculaire.

3. Procédé selon l'une des revendications 1 et 2, caractérisé en ce que les protéoglycanes solubles sont délipidés avant l'hydrolyse par le lysozyme.

4. Procédé selon la revendication 3, caractérisé en ce que la délipidation est effectuée par extraction à l'aide d'un ou plusieurs solvants des lipides.

5. Protéoglycanes bactériens comme ils peuvent être obtenus selon le procédé d'une des revendications 1 à 4 et qui présentent les caractéristiques suivantes :
— poids moléculaire compris entre                                     200 000 et 400 000,
— teneur en hexoses entre                                           8 et 10 %
— teneur en galactose entre                                       22 et 28 %
— teneur en hexosamines entre                                    3 et 6 %
— teneur en acides uroniques entre                              4 et 6 %
— teneur en protéines entre                                      35 et 50 %.

6. A titre de médicament, les protéoglycanes selon la revendication 5.

## Claims

1. Process for preparing bacterial membrane proteoglycans having an interferon-inducing action from soluble membrane proteoglycans of a non-encapsulated strain of Klebsiella pneumoniae, characterized in that:
— the crude membrane proteoglycans isolated from the said strain are treated in an aqueous medium with a base or a hypobromite and the aqueous phase containing the soluble proteoglycans is recovered,
— the soluble membrane proteoglycans are then hydrolysed with lysozyme, and
— the proteoglycans having a molecular weight of between 200,000 and 400,000 are separated from the hydrolysis product.

2. Process according to Claim 1, characterized in that the proteoglycans are separated by molecular sieve chromatography.

3. Process according to one of Claims 1 and 2, characterized in that the lipid is removed from the soluble proteoglycans before hydrolysis with lysozyme.

4. Process according to Claim 3, characterized in that the removal of lipid is accomplished by extraction using one or more lipid solvents.

5. Bacterial proteoglycans as can be obtained according to the process of one of Claims 1 to 4 and which possess the following characteristics :
— molecular weight between                                       200,000 and 400,000,
— hexose content between                                          8 and 10 %
— galactose content between                                       22 and 28 %
— hexosamine content between                                    3 and 6 %
— uronic acid content between                                     4 and 6 %
— protein content between                                          35 and 50 %.

6. By way of a medicinal product, the proteoglycans according to Claim 5.

## Patentansprüche

1. Verfahren zur Herstellung von bakteriellen Membranproteoglykanen mit interferoninduzierender Wirkung aus löslichen Membranproteoglykanen eines kapsellosen Klebsiella pneumoniae-Stammes, dadurch gekennzeichnet, daß :
— man in wässrigem Medium die nicht aufgearbeiteten, isolierten Membranproteoglykane des genannten Stammes mit einer Base oder einem Hypobromit behandelt und die die löslichen Proteoglykane enthaltende wäßrige Phase rückgewinnt,
— die löslichen Membranproteoglykane dann mit Hilfe von Lysozym hydrolisiert werden, und
— man die Proteoglykane mit einem Molekulargewicht zwischen 200 000 und 400 000 vom Hydrolyse-Produkt trennt.

2. Verfahren nach Anspruch 1, dadurch gekennzeichnet, daß die Proteoglykane durch Molekularsiebchromatographie getrennt werden.

3. Verfahren nach einem der Ansprüche 1 und 2, dadurch gekennzeichnet, daß die löslichen Proteoglykane vor der Hydrolyse durch Lysozym von Lipiden befreit werden.

4. Verfahren nach Anspruch 3, dadurch gekennzeichnet, daß die Lipidentfernung durch Extraktion mit Hilfe von einem oder mehreren Lipidlösenden Mitteln erfolgt.

5. Bakterielle Proteoglykane, wie sie gemäß dem verfahren nach einem der Ansprüche 1 bis 4 hergestellt werden können und die die folgenden Eigenschaften aufweisen :
— ein Molekulargewicht zwischen 200 000 und 400 000,
— einen Hexosegehalt zwischen 8 und 10 %
— einen Galaktosegehalt zwischen 22 und 28 %
— einen Hexosamingehalt zwischen 3 und  6 %
— einen Uronsäuregehalt zwischen 4 und  6 %
— einen Proteingehalt zwischen 35 und 50 %.
6. Die Proteoglykane nach Anspruch 5 als Arzneimittel.

FIG_1

FIG_2

% LYSE

60

40

20

10

200:1 100:1 50:1 25:1

1    3    6    9    15    18    21    R

t JOURS

EP 0 089 266 B2

FIG_3

FIG_4